# EUROPEAN PATENT SPECIFICATION

(11) **EP 1 721 886 B1**
(45) Date of publication and mention of the grant of the patent: **09.03.2011**
(21) Application number: 04792201.8
(22) Date of filing: 08.10.2004
(51) Int. Cl.: C07C 67/58, C07C 69/54, C02F 1/58, B01D 9/02

(54) **METHOD OF PROCESSING METHACRYLIC-ESTER-CONTAINING SOLUTION**
VERFAHREN ZUR VERARBEITUNG EINER METHACRYLSÄUREESTERHALTIGEN LÖSUNG
PROCEDE DE TRAITEMENT D'UNE SOLUTION CONTENANT DE L'ESTER METHACRYLIQUE

(30) Priority: 01.03.2004 JP 2004056333
(43) Date of publication of application: 15.11.2006
(73) Proprietor: Mitsubishi Chemical Corporation, Minato-ku Tokyo 108-0014 (JP)
(72) Inventor: YADA, Shuhei, MITSUBISHI CHEMICAL CORPORATION, Yokkaichi-shi, Mie 5108530 (JP); TAKASAKI, Kenji, MITSUBISHI CHEMICAL CORPORATION, Yokkaichi-shi, Mie 5108530 (JP); SUZUKI, Yoshiro, MITSUBISHI CHEMICAL CORPORATION, Yokkaichi-shi, Mie 5108530 (JP); OGAWA, Yasushi, MITSUBISHI CHEMICAL CORPORATION, Yokkaichi-shi, Mie 5108530 (JP)
(74) Representative: Woods, Geoffrey Corlett
(86) International application number: PCT/JP2004/014932
(87) International publication number: WO 2005/082831

(56) References cited:
- EP-A1- 0 618 187
- WO-A-03/022793
- JP-A- 62 099 345

## Description

### TECHNICAL FIELD

The present invention relates to a method for handling a (meth)acrylic ester-containing solution, and more particularly, to an improved method for handling a (meth)acrylic ester-containing solution which is treated by at least one process selected from washing, neutralization and extraction.

### BACKGROUND ARTS

(Meth)acrylic esters are produced by subjecting (meth)acrylic acid and alcohol to esterification reaction in the presence of a homogeneous acid catalyst or a heterogeneous solid acid catalyst. In the case of using the homogeneous acid catalyst, the resultant esterification reaction solution is washed and/or neutralized with an aqueous alkali solution after completion of the reaction to separate and remove the acid catalyst as well as unreacted (meth)acrylic acid from the reaction product. Also, in the case of using the heterogeneous solid acid catalyst, the resultant esterification reaction solution is extracted with water or an aqueous inorganic salt solution after completion of the reaction to separate and remove unreacted alcohol and unreacted (meth)acrylic acid from the reaction product. Further, the reaction mixtures obtained from the respective treatments are generally subjected to liquid-liquid extraction procedure to separate these mixtures into an organic phase containing the (meth)acrylic esters and a water phase containing organic acid salts, inorganic acid salts and/or alcohol.

Conventionally, the above water phase has been further distilled to recover effective ingredients therefrom. Waste water or a waste aqueous solution obtained after recovering the effective ingredients from the water phase has been directly recycled as the water, the aqueous alkali solution or the aqueous inorganic salt solution to the above respective treatments and reused therein.

However, when the waste water or waste aqueous solution discharged from the distillation column is directly recycled and reused, sludge is formed upon the liquid-liquid extraction procedure conducted after the respective treatments. The thus formed sludge tends to cause problems such as deterioration in liquid-liquid separation efficiency and insufficient phase separation.

Further, owing to formation of such a sludge, trays or packing materials used in distillation columns such as low-boiling component-removing column or alcohol recovery column, tend to suffer from deposition and clogging of solids, resulting in problems such as deteriorated distillation efficiency, increase in pressure loss, damage to rotating devices such as pump, and increase in load applied to distillation systems. These problems further lead to deterioration in quality of resultant products or poor productivity, so that the operation of the columns must be stopped to overhaul and clean the columns or replace the respective devices with new ones. As a result, the above conventional method has failed to achieve a continuous stable operation of the system for a long period of time, and, therefore, tends to be deteriorated in quality of products and unit requirement.

As a method of preventing formation of the sludge, there has been proposed the method of separating and removing insoluble components from an organic liquid by applying an ultrasonic wave thereto (for example, refer to Japanese Patent Application Laid-open (KOKAI) No. 8-10508). However, this method not only requires installation of additional devices, but also fails to exhibit sufficient effects.

In addition, there has also been proposed the method of installing a filter in a line conduit through which an organic liquid is flowed, to separate and remove insoluble components from the liquid (for example, refer to Japanese Patent Application Laid-open (KOKAI) No. 2003-231665). This method shows certain effects, but tends to suffer from formation of sludge and, therefore, is still unsatisfactory to ensure a continuous stable operation of the system.

### DISCLOSURE OF THE INVENTION

### PROBLEM TO BE SOLVED BY THE INVENTION

The present invention has been made in view of the above conventional problems. An object of the present invention is to provide a method for handling a (meth)acrylic ester-containing solution, in which the (meth)acrylic ester-containing solution is treated by at least one process selected from washing, neutralization and extraction, wherein said method is capable of ensuring a stable continuous operation of the treating system for a long period of time without deteriorating a liquid-liquid separation efficiency and a distillation efficiency in subsequent steps while inhibiting the formation of sludge, when waste water or a waste aqueous solution obtained by recovering effective ingredients from the (meth)acrylic ester-containing solution treated by the said at least one process is recycled to the previous process and reused therein.

### MEANS FOR SOLVING THE PROBLEM

As a result of the present inventors' earnest study for solving the above problems, it has been found that the formation of sludge upon the liquid-liquid separation procedure is caused by soluble components contained in the waste water or waste aqueous solution to be recycled to the previous process.

The present invention has been attained on the basis of the above finding as well as further studies. To accomplish the aim, in an aspect of the present invention, there is provided a method for handling a (meth)acrylic ester-containing solution by subjecting said (meth)acrylic ester-containing solution to at least one process selected from washing, neutralization and extraction, wherein waste water or a waste aqueous solution obtained by recovering effective ingredients from the (meth)acrylic ester-containing solution treated by the said at least one process is previously cooled to a temperature of 10 to 50°C and then solids are removed therefrom prior to recycling the waste water or waste aqueous solution to the previous process and reusing the same therein.

### EFFECT OF THE INVENTION

Thus, according to the present invention, in a method for handling a (meth)acrylic ester-containing solution in which the (meth)acrylic ester-containing solution is treated by at least one process selected from washing, neutralization and extraction, when waste water or a waste aqueous solution obtained by recovering effective ingredients from the (meth)acrylic ester-containing solution treated by the said at least one process is recycled to the previous process and reused therein, a stable continuous operation of the treating system can be ensured for a long period of time without deteriorating a liquid-liquid separation efficiency and a distillation efficiency in subsequent steps while inhibiting the formation of sludge.

### BRIEF DESCRIPTION OF THE DRAWING

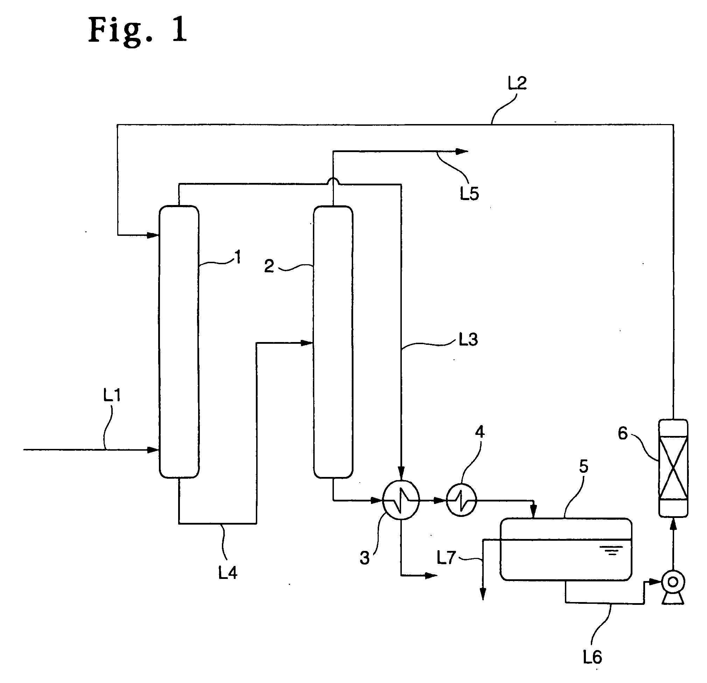

Fig. 1 is a flow diagram showing an embodiment of an extraction treatment according to the present invention.

### EXPLANATION OF REFERENCE NUMERALS

1: Extraction column; 2: Alcohol recovery column; 3: Heat exchanger; 4: Heat exchanger; 5: Buffer drum; 6: Filter

### PREFERRED EMBODIMENT FOR CARRYING OUT THE INVENTION

The present invention is described in detail below. The (meth)acrylic ester-containing solution to be handled according to the present invention is a reaction solution obtained by subjecting (meth)acrylic acid and alcohol to esterification reaction in the presence of an acid catalyst. The (meth)acrylic ester as the esterification reaction product is not particularly restricted, and examples of the (meth)acrylic ester may include methyl (meth)acrylate, ethyl (meth)acrylate, propyl (meth)acrylate, butyl (meth)acrylate, cyclohexyl (meth)acrylate, 2-ethylhexyl (meth)acrylate and methoxyethyl (meth)acrylate. As the alcohol, there may be used alcohols suitable as raw materials of the above (meth)acrylic esters. From such a viewpoint that esters produced from alcohols having a smaller number of carbon atoms are more effectively extracted with alcohol, the handling method of the present invention can be more advantageously applied to treatments of methyl acrylate, ethyl acrylate and methyl methacrylate.

Examples of the acid catalyst may include homogeneous acid catalysts and heterogeneous solid acid catalysts. Specific examples of the homogeneous acid catalysts may include sulfuric acid, p-toluenesulfonic acid and methanesulfonic acid. The homogeneous acid catalysts are generally contained in a water phase obtained after subjecting the esterification reaction solution to at least one treatment selected from washing, neutralization and extraction with water and/or an aqueous alkali solution. Specific examples of the heterogeneous solid acid catalysts may include strong acid cation exchange resins, activated clay and acidic zeolite. The unreacted (meth)acrylic acid or unreacted alcohol contained in the esterification reaction product are generally contained in the water phase obtained after subjecting the esterification reaction solution to at least one treatment selected from washing, neutralization and extraction with water and/or an aqueous alkali solution.

The esterification reaction is not particularly restricted, and may be conducted by conventionally known methods. The industrial process for production of the (meth)acrylic esters may be conducted by either a batch method or a continuous method. In the esterification reaction, the molar ratio between the raw materials, the kind and amount of catalyst used in the reaction, the reaction methods and the reaction conditions may be appropriately selected according to the kind of alcohol used therein. Also, in order to prevent the (meth)acrylic acid and the (meth)acrylic ester as the reaction product from being polymerized upon the reaction and distillation, an oxygen-containing gas as a polymerization inhibitor or a polymerization terminator is preferably added in the reactor, the distillation column or the like.

The (meth)acrylic ester-containing solution obtained by the esterification reaction is subjected to various processes such as separation of catalysts, concentration and purification. The respective processes include treatments as unit procedures such as washing, neutralization, extraction, evaporation and distillation. Examples of water or an aqueous solution used for the washing, neutralization and extraction treatments may include, in addition to the above water and/or aqueous alkali solution, aqueous solutions of inorganic salts such as ammonium sulfate and sodium sulfate, and solutions containing catalysts used in the esterification reaction or salts (inorganic acid salts and organic acid salts) of (meth)acrylic acid for the purposes of more effectively extracting alcohols into the water phase or facilitating separation into the organic phase and the water phase due to the difference in specific gravity therebetween.

Examples of the apparatuses used for the washing, neutralization and extraction treatments may include various apparatuses such as mixer/settler-type apparatuses, extraction column-type apparatuses and stirring tank-type apparatuses, though not particularly limited thereto. In particular, the washing and/or extraction treatments are preferably conducted using an extraction column. The type of the extraction column may include a packed tower type, a tray tower type, a rotary disk type, etc.

The mixtures obtained from the respective treatments are generally separated by liquid-liquid extraction procedure into an organic phase containing the (meth)acrylic ester and a water phase containing organic salts, inorganic salts and/or alcohols. The liquid-liquid extraction procedure may be generally conducted at a low temperature to attain a high extraction efficiency and a high separation efficiency. In addition, from the viewpoint that the (meth)acrylic ester is susceptible to hydrolysis reaction, the low-temperature liquid-liquid extraction procedure is also preferable. The liquid-liquid extraction procedure may be usually conducted at a temperature of 10 to 50°C.

After recovering effective ingredients such as catalysts, (meth)acrylic acid, alcohols and (meth)acrylic esters from the water phase by a distillation procedure, a large part of the resultant waste water or waste aqueous solution is recycled to the previous process and reused therein. The temperature of the waste water or waste aqueous solution to be recycled and reused is usually in the range of 80 to 110°C since it has been subjected to the distillation procedure.

Meanwhile, the (meth)acrylic ester-containing solution to be subjected to the above washing, neutralization and extraction treatments may include, in addition to the esterification reaction product itself, liquids obtained by removing a large part of the catalysts and/or (meth)acrylic acid therefrom by distillation, extraction, etc.

In the present invention, it is important that the waste water or waste aqueous solution is previously cooled to a temperature of 10 to 50°C, preferably 10 to 40°C to precipitate solids, and then the thus precipitated solids are removed therefrom in the above temperature range, prior to recycling the waste water or waste aqueous solution to the previous process and reusing the same therein.

The above temperature range corresponds to that used in the liquid-liquid extraction procedure. When the waste water or waste aqueous solution is cooled to the above temperature range, a solubility of soluble components contained therein can be reduced, resulting in precipitation of the soluble components. Therefore, the reuse of the waste water or waste aqueous solution subjected to these treatments can prevent precipitation of solids upon the liquid-liquid extraction procedure, thereby enabling a stable long-term continuous operation of the treating system without deterioration in liquid-liquid separation efficiency as well as deterioration in distillation efficiency in the subsequent steps.

When the temperature of the waste water or waste aqueous solution upon removal of the solids is more than 50°C, the soluble components may fail to be precipitated sufficiently, so that solids tend to be precipitated upon the liquid-liquid extraction procedure. On the other hand, when the waste water or waste aqueous solution is excessively cooled to a temperature of less than 10°C, energy loss tends to be caused.

Examples of the solids removed from the waste water or waste aqueous solution may include poly(meth)acrylic acid, poly(meth)acrylic esters, high-molecular compounds such as Michael adduct-type high-molecular polymers, which are produced during the process for production of (meth)acrylic esters, and various salts added for the purpose of facilitating the liquid-liquid extraction procedure. In addition, examples of the other solids may include sludge such as iron rust, dregs, colloidal sediment, scum, etc.

The thus precipitated solids are separated from the waste water or waste aqueous solution using a filtering means or a precipitation separation means. Examples of the filtering means used for separating the solids may include strainers, filters, etc., and examples of the precipitation separation means used for separating the solids may include stationary separators, centrifugal separators, etc., though not particularly limited thereto. From the standpoints of separation efficiency, installation costs and facilitated operation, the above solids is preferably separated from the waste water or waste aqueous solution using a filter capable of penetrating particles smaller than those having a particle size of 1 to 10 µm therethrough. Meanwhile, the separation procedure for separating the solids using the stationary separation tank or filter may also be conducted at the same temperature range of 10 to 50°C as used upon cooling and precipitating the solids.

A preferred embodiment of the present invention is explained by referring to the flow diagram shown in Fig. 1. Meanwhile, in this embodiment, the extraction treatment of methyl acrylate is illustrated. A methyl acrylate-containing solution is continuously fed into an extraction column (1) from a lower portion thereof through a line (L1). A waste aqueous solution recycled from the preceding process, which is used in the extraction treatment is passed through a filter (6) to remove solids therefrom, and then continuously fed to an upper portion of the extraction column (1) through a line (L2). An organic phase as a raffinate obtained by removing mainly methanol from the solution by extraction is withdrawn from the upper portion of the extraction column (1) through a line (L3), and then fed to a low-boiling component separation step (not shown) through a heat exchanger (3). A water phase as an extract containing mainly methanol as extracted is withdrawn from a lower portion of the extraction column (1) through a line (L4), and then continuously fed to an alcohol recovery column (2). In the alcohol recovery column (2), methanol is recovered from an upper portion thereof, and then recycled to a reaction step (not shown) through a line (L5) and reused therein. The waste aqueous solution containing a small amount of organic substances (such as polymers) and inorganic substances (such as iron rust) which is withdrawn from a bottom of the alcohol recovery column is cooled by heat exchangers (3) and (4), and temporarily stored in a buffer drum (5) in which some solids are precipitated in the waste aqueous solution while precipitated light solids are floated and raised on a surface of the solution and coagulated together. These precipitated light solids are discharged through a line (L7). The waste aqueous solution from which the light precipitated solids are removed is fed through a line (L6) to the above filter (6) where fine precipitates as scum in the solution are removed therefrom, and then recycled to the upper portion of the extraction column (1) for reuse therein.

### EXAMPLES

The present invention is described in more detail by Examples, but the Examples are only illustrative and not intended to limit the scope of the present invention.

### Example 1:

A raw fraction obtained by distilling off heavy components such as acrylic acid from the esterification reaction product produced in the process for production of methyl acrylate was continuously treated using the apparatuses as shown in the flow diagram of Fig. 1.

A methyl acrylate-containing solution was continuously fed into a packed tower-type extraction column (1) from a lower portion thereof through a line (L1). The raw material fed to the extraction column (1) had an average composition containing 13% by weight of water, 10% by weight of methanol, 75% by weight of methyl acrylate and 2% by weight of others, and was fed at an average flow rate of 2.8 tons/h. Also, the extraction procedure was conducted at a temperature of 25°C.

A water phase as an extract containing mainly methanol as extracted was withdrawn from the lower portion of the extraction column (1) through a line (L4), and then continuously fed to an alcohol recovery column (2) operated under ordinary pressure. In the alcohol recovery column (2), methanol was recovered from a top thereof, and then recycled to a reaction step (not shown) through a line (L5) and reused therein. A waste aqueous solution containing a small amount of organic substances (such as polymers) and inorganic substances (such as iron rust), which were withdrawn from a bottom of the alcohol recovery column was cooled from 98°C to 25°C by heat exchangers (3) and (4), and then temporarily stored in a buffer drum (5). After removing light solids from the waste aqueous solution containing precipitated solids through a line (L7), the waste aqueous solution was fed through a line (L6) to a filter (6) (cartridge filter capable of penetrating particles having a particle size of 3 µm or less) where residual solids were separated from the solution, and then recycled to the upper portion of the extraction column (1) and reused therein.

Meanwhile, the organic phase as a raffinate obtained by extracting and removing mainly methanol from the solution was withdrawn from the upper portion of the extraction column (1) through a line (L3) and then fed to a low-boiling component separation step (not shown) through the heat exchanger (3).

As a result of conducting a continuous operation of the above treating system for 300 days, it was confirmed that the methanol concentration in the organic phase obtained from the upper portion of the extraction column (1) was stably kept within the range of 0.1 to 0.2% by weight, the differential pressure between the top and bottom of the alcohol recovery column (2) remained substantially unchanged, thereby achieving a stable continuous operation of the system.

### Example 2:

The same procedure as defined in Example 1 was conducted except that the raw material fed had an average composition containing 13% by weight of water, 10% by weight of methanol, 75% by weight of methyl acrylate and 2% by weight of others, and the average flow rate and the extraction temperature were changed to 3.0 tons/h and 20°C, respectively, thereby conducting a continuous operation of the system. As a result of conducting the continuous operation for 300 days, it was confirmed that the methanol concentration in the organic phase obtained from the upper portion of the extraction column (1) was stably kept within the range of 0.2 to 0.3% by weight, thereby achieving a stable continuous operation of the system.

### Comparative Example 1:

The same procedure as defined in Example 1 was conducted using the same apparatuses, raw materials and operation conditions as in Example 1 except that the waste aqueous solution discharged from the bottom of the alcohol recovery column (2) was not subjected to the cooling and filtering treatments, thereby conducting a continuous operation of the system. As a result, from the time at which 200 days elapsed, the methanol concentration in the organic phase obtained form the upper portion of the alcohol recovery column (2) exceeded 0.2% by weight. Further, accumulation of precipitated solids in the extraction column (1) was initiated at that time, so that the liquid-liquid interface therein became unstable. In addition, the amounts of ethanol and water in the liquid flowed through the line (L3) was increased, so that it became impossible to continue the operation of facilities subsequent to the line (L3). On the other hand, in the alcohol recovery column (2), increase in differential pressure between the top and bottom thereof was initiated. Further, since the bottom temperature of the alcohol recovery column (2) was raised, the amount of steam fed as a heating medium for the alcohol recovery column (2) was lowered. As a result, it became impossible to continue a stable operation of the system.

### Comparative Example 2:

The same procedure as defined in Example 2 was conducted using the same apparatuses, raw materials and operation conditions as in Example 2 except that the waste aqueous solution discharged from the bottom of the alcohol recovery column (2) was not subjected to the cooling and filtering treatments, thereby conducting a continuous operation of the system. As a result, from the time at which 150 days elapsed, the methanol concentration in the organic phase obtained form the upper portion of the alcohol recovery column (2) exceeded 0.3% by weight. Further, accumulation of precipitated solids in the extraction column (1) was initiated at that time, so that the liquid-liquid interface therein became unstable. In addition, the amounts of ethanol and water in the liquid flowed through the line (L3) was increased, so that it became impossible to continue the operation of facilities subsequent to the line (L3). On the other hand, in the alcohol recovery column (2), increase in differential pressure between the top and bottom thereof was initiated. Further, since the bottom temperature of the alcohol recovery column (2) was raised, the amount of steam fed as a heating medium for the alcohol recovery column (2) was lowered. As a result, it became impossible to continue a stable operation of the system.

## Claims

1. A method for handling a (meth)acrylic ester-containing solution by subjecting said (meth)acrylic ester-containing solution to at least one process selected from washing, neutralization and extraction, wherein waste water or a waste aqueous solution obtained by recovering effective ingredients from the (meth)acrylic ester-containing solution treated by said at least one process is previously cooled to a temperature of 10 to 50°C and then solids are removed therefrom prior to recycling the waste water or waste aqueous solution to the previous process and reusing the same therein.

2. A method according to claim 1, wherein the solids is removed by a filtering means or a precipitation separation means.

3. A method according to claim 1 or 2, wherein the washing and/or extraction processes are conducted using an extraction column.

4. A method according to any of claims 1 to 3, wherein the (meth)acrylic ester is methyl acrylate, ethyl acrylate or methyl methacrylate.

5. The method of any one of the preceding claims, wherein the waste water or waste aqueous solution is obtained by recovering effective ingredients from the (meth)acrylic ester-containing solution by a distillation procedure, and the waste water or waste aqueous solution has a temperature in the range of 80 to 110°C.

## Patentansprüche

1. Verfahren zur Handhabung einer (Meth)acrylsäureester enthaltenden Lösung, in dem die (Meth)acrylsäureester enthaltende Lösung mindestens einem Prozess, ausgewählt aus Waschen, Neutralisation und Extraktion, unterzogen wird, worin Abwasser oder eine wässrige Abfallösung, das/die durch Zurückgewinnen der wirksamen Inhaltsstoffe aus der (Meth)acrylsäureester enthaltenden Lösung, die mit dem mindestens einen Prozess behandelt wird, erhalten wird, vorher auf eine Temperatur von 10 bis 50°C gekühlt wird und dann die Feststoffe hieraus vor dem Recyceln des Abwassers oder der wässrigen Abfallösung in den vorhergehenden Prozess entfernt werden und diejenige hierin wiederverwendet wird.

2. Verfahren gemäss Anspruch 1, worin die Feststoffe durch Filtermittel oder durch ein Ausfällungs-Abtrennungs-Mittel entfernt werden.

3. Verfahren gemäss Anspruch 1 oder 2, worin die Wasch- und/oder Extraktionsprozesse unter Verwendung einer Extraktionssäule durchgeführt werden.

4. Verfahren gemäss irgendeinem der Ansprüche 1 bis 3, worin der (Meth)acrylsäureester Methylacrylat, Ethylacrylat oder Methylmethacrylat ist.

5. Verfahren gemäss irgendeinem der vorhergehenden Ansprüche, worin das Abwasser oder die wässrige Abfallösung durch Zurückgewinnen der wirksamen Inhaltsstoffe aus der (Meth)acrylsäureester enthaltenden Lösung durch eine Destillationsprozedur erhalten ist und das Abwasser oder die wässrige Abfallösung eine Temperatur im Bereich von 80 bis 110°C aufweist.

## Revendications

1. Procédé pour traiter une solution contenant un ester (méth)acrylique en soumettant ladite solution contenant un ester (méth)acrylique à au moins un procédé choisi parmi un lavage, une neutralisation et une extraction, dans lequel les eaux usées ou une solution aqueuse usée obtenue par récupération de composants efficaces à partir de la solution contenant un ester (méth)acrylique traitée par ledit au moins un procédé sont préalablement refroidies à une température de 10 à 50 °C et ensuite les matières solides sont enlevées avant de recycler les eaux usées ou la solution aqueuse usée dans le procédé précédent et de réutiliser celles-ci dans ledit procédé.

2. Procédé selon la revendication 1, dans lequel les matières solides sont enlevées par un moyen de filtration ou un moyen de séparation par précipitation.

3. Procédé selon la revendication 1 ou 2, dans lequel les procédés de lavage et/ou d'extraction sont conduits en utilisant une colonne d'extraction.

4. Procédé selon l'une quelconque des revendications 1 à 3, dans lequel l'ester (méth)acrylique est l'acrylate de méthyle, l'acrylate d'éthyle ou le méthacrylate de méthyle.

5. Procédé de l'une quelconque des revendications précédentes, dans lequel les eaux usées ou la solution aqueuse usée sont obtenues par récupération de composants efficaces à partir de la solution contenant un ester (méth)acrylique par une procédure de distillation, et les eaux usées ou la solution aqueuse usée ont une température dans la plage de 80 à 110 °C..
